# EUROPÄISCHE PATENTSCHRIFT

(11) **EP 0 414 078 B1**
(45) Veröffentlichungstag und Bekanntmachung des Hinweises auf die Patenterteilung: **01.06.1994**
(21) Anmeldenummer: 90115444.3
(22) Anmeldetag: 11.08.1990
(51) Int. Cl.: C07D 215/14, A61K 31/47

(54) **Disubstituierte (Chinolin-2-yl-methoxy) phenylessigsäure-Derivate**
Disubstituted (quinolin-2-yl-methoxy)-phenylacetic acid derivatives
Dérivés disubstitués de l'acide (quinoléine-2-yl-méthoxy)-phénylacétique

(30) Priorität: 24.08.1989 DE 3927931
(43) Veröffentlichungstag der Anmeldung: 27.02.1991
(73) Patentinhaber: BAYER AG, 51368 Leverkusen (DE)
(72) Erfinder: Mohrs, Klaus, Dr., D-5600 Wuppertal 1 (DE); Raddatz, Siegfried, Dr., D-5000 Köln 80 (DE); Fruchtmann, Romanis, D-5000 Köln 1 (DE); Kohlsdorfer, Christian, Dr., D-5042 Erftstadt (DE); Müller-Peddinghaus, Reiner, Prof., D-5060 Bergisch-Gladbach 2 (DE); Theisen-Popp, Pia, Dr., D-5060 Bergisch-Gladbach 2 (DE)

(56) Entgegenhaltungen:
- EP-A- 0 181 568
- EP-A- 0 339 416
- EP-A- 0 344 519
- EP-A- 0 349 062
- US-A- 4 661 499
- CHEMICAL ABSTRACTS, Band 106, Nr. 25, 22. Juni 1987, Seite 44, Zusammenfassung-Nr. 207457g, Columbus, Ohio, US; C.M. TENNANT et al.: "Effects of a 5-lipoxygenase inhibitor, REV-5901, on leukotriene and histamine release from human lung tissue in vitro"
- CHEMISCHE BERICHTE, Band 120, Nr. 4, April 1987, Seiten 649-651; G.E. JEROMIN et al.: "Seitenkettenchlorierungen von N-Heterocyclen mit Trichlorisocyanursäure"
- JOURNAL OF THE CHEMICAL SOCIETY/CHEMICAL COMMUNICATIONS, 7. Juni 1972, Seite 668; T. INOUE et al.: "A new photo-fries rearrangement of methyl aryl oxalates"

## Beschreibung

Die Erfindung betrifft disubstituierte (Chinolin-2-yl-methoxy)phenylessigsäure-Derivate, Verfahren zu ihrer Herstellung und ihre Verwendung in Arzneimitteln.

Es ist bekannt, daß 3-(Chinolin-2-yl-methoxy)phenylessigsäure und 2-[3-(Chinolin-2-yl-methoxy)phenyl]propionsäure und deren Methyl- und Ethylester eine antiinflammatorische und antiallergische Wirkung besitzen [vgl. EP-A 181 568].

Es wurden nun disubstituierte (Chinolin-2-yl-methoxy)phenylessigsäure-Derivate der allgemeinen Formel (I) 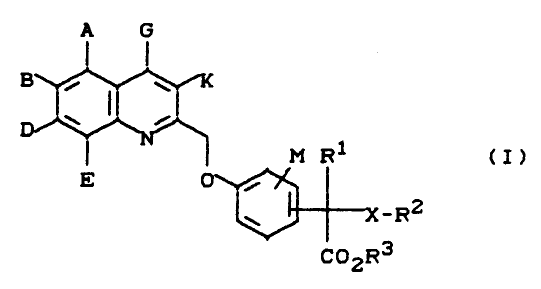
in welcher
- A, B, D, E, G, K und M: gleich oder verschieden sind und
- für Wasserstoff, Hydroxy, Halogen, Trifluormethyl, Trifluormethoxy oder Carboxy stehen,
- für geradkettiges oder verzweigtes Alkyl mit bis zu 10 Kohlenstoffatomen stehen, das gegebenenfalls durch Hydroxy oder Halogen substituiert ist,
- für geradkettiges oder verzweigtes Alkoxy oder Alkoxycarbonyl mit bis zu 10 Kohlenstoffatomen stehen, oder
- für Aryl mit 6 bis 10 Kohlenstoffatomen stehen, das gegebenenfalls durch Halogen, Nitro, Cyano oder durch geradkettiges oder verzweigtes Alkyl oder Alkoxy mit bis zu 8 Kohlenstoffatomen substituiert ist,
- R¹: - für geradkettiges oder verzweigtes Alkyl mit bis zu 10 Kohlenstoffatomen steht, das gegebenenfalls durch Halogen oder durch Cycloalkyl mit 3 bis 8 Kohlenstoffatomen substituiert ist, oder
- für Cycloalkyl mit 3 bis 8 Kohlenstoffatomen steht, das gegebenenfalls durch Halogen oder geradkettiges oder verzweigtes Alkyl mit bis zu 8 Kohlenstoffatomen substituiert ist,
- R²: - für Wasserstoff, geradkettiges oder verzweigtes Alkyl mit bis zu 10 Kohlenstoffatomen oder für Aryl mit 6 bis 10 Kohlenstoffatomen steht,
- R³: - für Wasserstoff, geradkettiges oder verzweigtes Alkyl mit bis zu 10 Kohlenstoffatomen oder für Aryl mit 6 bis 10 Kohlenstoffatomen steht,
- X: - für Sauerstoff oder Schwefel steht,
und deren Salze gefunden.

Im Rahmen der vorliegenden Erfindung werden physiologisch unbedenkliche Salze bevorzugt. Physiologisch unbedenkliche Salze der disubstituierten (Chinolin-2-yl-methoxy)phenylessigsäure-Derivate können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Salze der einwertigen Metalle wie Alkalimetalle und die Ammoniumsalze. Bevorzugt werden Natrium-, Kalium- und Ammoniumsalze.

Bevorzugt sind Verbindungen der allgemeinen Formel (I),
in welcher
- A, B, D, E, G, K und M: gleich oder verschieden sind und
- für Wasserstoff, Fluor, Chlor, Trifluormethoxy oder Carboxy stehen,
- für geradkettiges oder verzweigtes Alkyl mit bis zu 8 Kohlenstoffatomen stehen, das gegebenenfalls durch Hydroxy, Fluor, Chlor oder Brom substituiert ist,
- für geradkettiges oder verzweigtes Alkoxy oder Alkoxycarbonyl mit bis zu 8 Kohlenstoffatomen stehen, oder
- für Phenyl stehen, das gegebenenfalls durch Fluor, Chlor, Brom, Nitro, Cyano oder durch geradkettiges oder verzweigtes Alkyl oder Alkoxy mit bis zu 6 Kohlenstoffatomen substituiert ist,
- R¹: - für geradkettiges oder verzweigtes Alkyl mit bis zu 8 Kohlenstoffatomen steht, das gegebenenfalls durch Fluor, Chlor, Brom, Cyclopropyl, Cyclobutyl, Cyclopentyl, Cyclohexyl oder Cycloheptyl substituiert ist, oder
- für Cyclopropyl, Cyclobutyl, Cyclopentyl, Cyclohexyl oder Cycloheptyl steht, das gegebenenfalls durch Fluor, Chlor oder geradkettiges oder verzweigtes Alkyl mit bis zu 6 Kohlenstoffatomen substituiert ist,
- R²: - für Wasserstoff, geradkettiges oder verzweigtes Alkyl mit bis zu 8 Kohlenstoffatomen oder für Phenyl steht,
- R³: - für Wasserstoff, geradkettiges oder verzweigtes Alkyl mit bis zu 8 Kohlenstoffatomen oder Phenyl steht,
- X: - für Sauerstoff oder Schwefel steht
und deren Salze.

Besonders bevorzugt sind Verbindungen der allgemeinen Formel (I),
in welcher
- A, B, D, E, G, K und M: gleich oder verschieden sind und
- für Wasserstoff, Fluor, Chlor, geradkettiges oder verzweigtes Alkyl oder Alkoxy mit bis zu 6 Kohlenstoffatomen stehen,
- R¹: - für geradkettiges oder verzweigtes Alkyl mit bis zu 6 Kohlenstoffatomen steht, das gegebenenfalls durch Fluor, Cyclopropyl, Cyclobutyl, Cyclopentyl, Cyclohexyl oder Cycloheptyl substituiert ist, oder
- für Cyclopropyl, Cyclobutyl, Cyclopentyl, Cyclohexyl oder Cycloheptyl steht, das gegebenenfalls durch Fluor oder durch geradkettiges oder verzweigtes Alkyl mit bis zu 4 Kohlenstoffatomen substituiert ist,
- R²: - für Wasserstoff oder für geradkettiges oder verzweigtes Alkyl mit bis zu 6 Kohlenstoffatomen oder für Phenyl steht,
- R³: - für Wasserstoff oder für geradkettiges oder verweigtes Alkyl mit bis zu 6 Kohlenstoffatomen steht,
- X: - für Sauerstoff steht
und deren Salze.

Ganz besonders bevorzugt sind Verbindungen der allgemeinen Formel (I), in denen die Chinolylmethoxygruppierung am Phenyl in 4-Stellung zum substitutierten Essigsäurerest steht.

Außerdem wurde ein Verfahren zur Herstellung der erfindungsgemäßen Verbindungen der allgemeinen Formel (I), 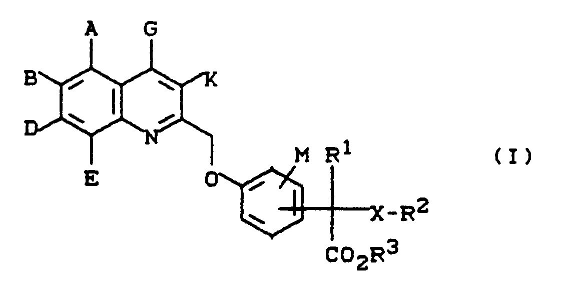
in welcher
- A, B, D, E, G, K, M, R¹, R², R³ und X: die oben angegebene Bedeutung haben
gefunden, das dadurch gekennzeichnet ist, daß man
Ketoester der allgemeinen Formel (II) 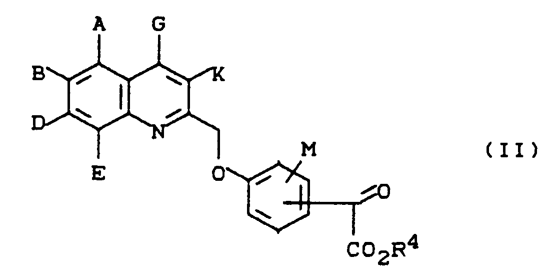
in welcher
- A, B, D, E, G, K und M: die oben angegegebene Bedeutung haben,
und
- R⁴: - die oben angegebene Bedeutung von R³ hat, aber nicht für Wasserstoff steht,
zunächst mit Grignard- oder metallorganischen Verbindungen der allgemeinen Formeln (III) und (IIIa)

R¹-Y (III)

(R¹)ₙ-Y (III)

in welcher
- R¹: die oben angegebene Bedeutung hat
- n: die Zahl 2 oder 3 bedeutet,
und
- Y: - für den typischen Grignardrest Z-W steht, worin
   Z - Magnesium, Cadmium oder Zink bedeutet
   und
   W - Chlor, Brom oder Jod bedeutet,
   oder
- für Lithium, Natrium, Magnesium, Aluminium, Cadmium oder Zink steht,
in inerten Lösemitteln reduziert und unter anschließender Abspaltung der Gruppe Y zu Verbindungen der allgemeinen Formel (Ia) 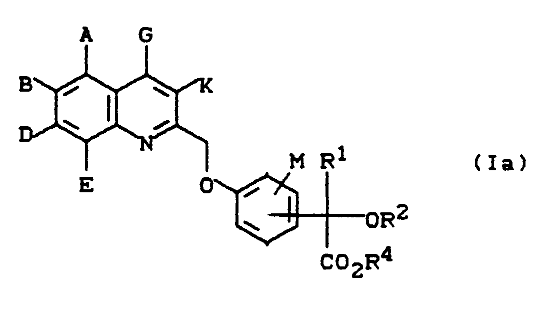
in welcher
- A, B, D, E, G, K, M, R¹, R² und R⁴: die oben angegebene Bedeutung haben,
umsetzt,
und im Fall, daß X für Schwefel steht, die Verbindungen der Formel (Ia) nach üblicher Methode mit Thiolen der allgemeinen Formel (IV)

HS-R² (IV)

in welcher
- R²: die oben angegebene Bedeutung hat,
umsetzt,
und im Fall der Säuren in einem letzten Schritt die Ester alkalisch verseift.

Das Verfahren kann durch folgendes Formelschema erläutert werden: 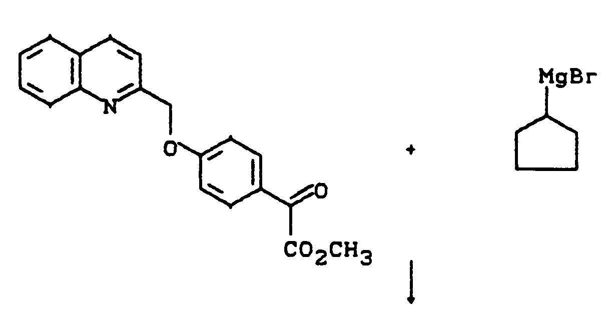 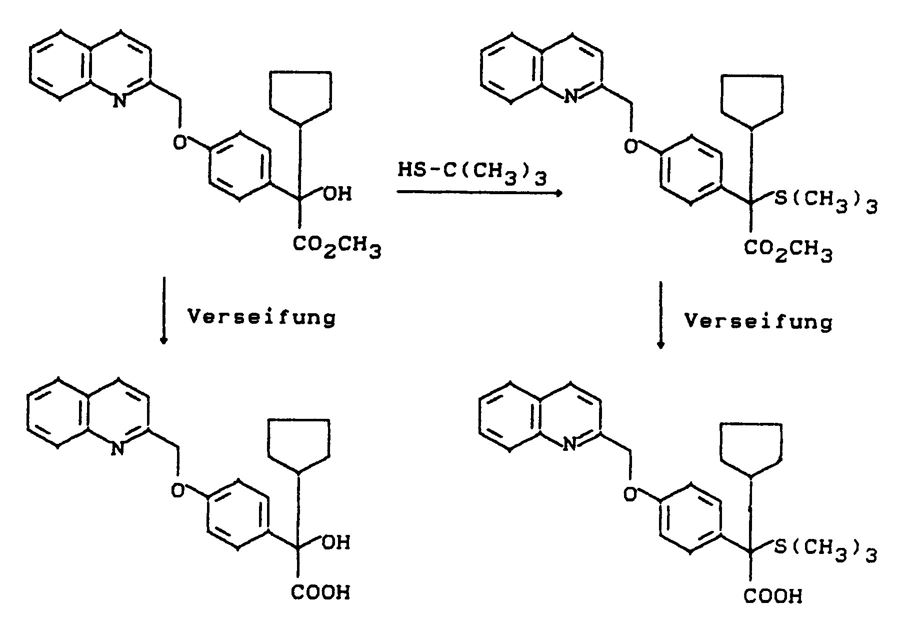
Als Lösemittel für die Reduktion eignen sich die üblichen oragnischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenstoffe wie Benzol, Toluol, Xylol, Hexan, Cyclohexan, oder Erdölfraktionen oder Dimethylformamid. Ebenso ist es möglich, Gemische der genannten Lösemittel zu verwenden. Bevorzugt sind Tetrahydrofuran und Diethylether.

Die Reduktion wird im allgemeinen in einem Temperaturbereich von -80°C bis +30°C, bevorzugt bei -40°C bis +25°C durchgeführt.

Die Reduktion wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Abspaltung der Gruppe Y erfolgt nach der für Grignard-Reaktionen üblichen Methode mit wäßriger Ammoniumchloridlösung [vgl. J. March, Advanced Organic Chemistry, Second Edition S. 836].

Die Verbindungen der allgemeinen Formeln (III) und (IIIa) sind an sich bekannt oder können nach üblicher Methode hergestellt werden [vgl. K. Nützel, Houben-Weyl, Methoden der organischen Chemie, 4. Aufl. Bd. 13/2a, 53 ff. (Thieme Verlag, Stuttgart) 1973; M.S. Kharash, O. Reinmuth, Grignard Reactions of Nonmetallic Compounds, Prentice Hall, New York, 1974; Uhlman XII, 370; Houben-Weyl XIII/2a, 289-302; R.I. Trust, R.E. Ireland, Org. Synth. 53, 116, (1973); O. Grummitt, E.I. Becker, Org. Synth. Coll. Vol. IV, 771 (1963); H. Adkins, W. Zartman, Org. Synth. Coll. Vol. II, 606 (1943)].

Im allgemeinen setzt man 1 bis 3 Mol, bevorzugt 1,1 Mol der Grignard Verbindungen bzw. der metallorganischen Verbindungen der allgemeinen Formeln (III) und (IIIa) bezogen auf 1 Mol Reaktionspartner ein.

Die Einführung einer Thiolo- oder Mercaptogruppe erfolgt in an sich bekannter Weise (Williamson-Synthese) in einem der oben aufgeführten inerten Lösemittel und vorzugsweise in einer Phasentransferreaktion [vgl. Synthesis 447, (1975); J. Am. Chem. Soc. 97, 2345 (1975); J. Am. Chem. Soc. 71, 84 (1949); J. Chem. Soc. 1694 (1962); Synthesis 818 (1974); Synthesis 430 (1974)].

Die Thiole der allgemeinen Formel (IV) sind an sich bekannt oder können nach üblicher Methode hergestellt werden [Beilstein 6, 8; 6(3), 1810, 6, 2, 4].

Die Ketoester der allgemeinen Formel (II) sind neu und können hergestellt werden, indem man
Verbindungen der allgemeinen Formel (V) 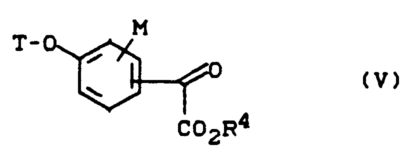
in welcher
- R⁴ und M: die oben angegebene Bedeutung haben
und
- T: - für eine typische Hydroxyschutzgruppe, wie beispielsweise Benzyl oder tert.Butyl steht,
mit Halogenmethylchinolinen der Formel (VI) 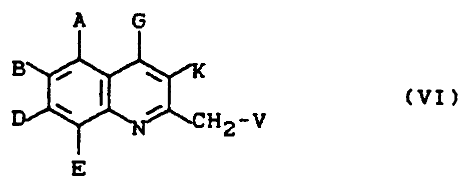
in welcher
- A, B, D, E, G und K: die oben angegebene Bedeutung haben
und
- V: - für Halogen steht,
nach Abspaltung der Schutzgruppe T in inerten Lösemitteln, gegebenenfalls in Anwesenheit einer Base verethert.

Die Abspaltung der Schutzgruppen aus den entsprechenden Ethern erfolgt nach üblicher Methode, beispielsweise durch hydrogenolytische Spaltung der Benzylether in den oben aufgeführten inerten Lösemitteln in Anwesenheit eines Katalysators mit Wasserstoff-Gas [vgl. außerdem Th. Greene: "Protective Groups in Organic Synthesis", J. Wiley & Sons, 1981, New York].

Die Veretherung kann in inerten organischen Lösemitteln gegebenenfalls in Anwesenheit einer Base durchgeführt werden.

Lösemittel für die Veretherung können inerte organische Lösemittel sein, die sich unter den Reaktionsbedingungen nicht verändern. Hierzu gehören vorzugsweise Ether wie beispielsweise Dioxan, Tetrahydrofuran oder Diethylether, Halogenkohlenwasserstoffe wie Dichlormethan, Trichlormethan, Tetrachlormethan, 1,2-Dichlorethan oder Trichlorethylen, Kohlenwasserstoffe wie Benzol, Xylol, Toluol, Hexan, Cyclohexan oder Erdölfraktionen, Nitromethan, Dimethylformamid, Acetonitril, Aceton oder Hexamethylphosphorsäuretriamid. Ebenso ist es möglich, Gemische der Lösemittel einzusetzen.

Als Basen für die Veretherung können anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder organische Amine (Trialkyl(C₁-C₆)amine) wie Triethylamin, oder Heterocyclen wie Pyridin, Methylpiperidin, Piperidin oder Morpholin.

Es ist auch möglich, als Basen Alkalimetalle wie Natrium und deren Hydride, wie Natriumhdyrid, einzusetzen.

Die Veretherung erfolgt im allgemeinen in einem Temperaturbereich von 0°C bis +150°C, bevorzugt von +10°C bis +100°C.

Die Veretherung wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Im allgemeinen setzt man 0,5 bis 5, bevorzugt 1 bis 2 Mol Halogenid, bezogen auf 1 Mol des Reaktionspartners ein. Die Base wird im allgemeinen in einer Menge von 0,5 bis 5 Mol, bevorzugt von 1 bis 3 Mol bezogen auf das Halogenid eingesetzt.

Die Verbindungen der allgemeinen Formel (V) sind bekannt oder können nach üblicher Methode hergestellt werden [vgl. Chem. Commun. 1972, (11), 668].

Die Verbindungen der allgemeinen Formel (VI) sind ebenfalls bekannt oder können nach üblicher Methode hergestellt werden [vgl. Chem. Ber. 120, 649 (1987)].

Die Verseifung der Carbonsäureester erfolgt nach üblichen Methoden, indem man die Ester in inerten Lösemitteln mit üblichen Basen behandelt.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C is +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol bezogen auf 1 Mol des Esters eingesetzt. Besonders bevorzugt verwendet man molare Mengen der Reaktanden.

Die Verbindungen der allgemeinen Formel (I) zeigen überraschenderweise eine hohe in vitro Aktivität als Leukotriensynthesehemmer und eine starke in vivo Wirkung nach oraler Applikation.

Die erfindungsgemäßen disubstituierten (Chinolin-2-yl-methoxy)phenylessigsäure-Derivate können als Wirkstoffe in Arzneimitteln eingesetzt werden. Die Stoffe können als Hemmer von enzymatischen Reaktionen im Rahmen des Arachidonsäurestoffwechsels, insbesondere der 5-Lipoxygenase, wirken.

Sie sind somit bevorzugt zur Behandlung und Verhütung von Erkrankungen der Atemwege wie Allergien/Asthma, Bronchitis, Emphysema, Schocklunge, pulmonaler Hypertonie, Entzündungen/Rheuma und Ödemen, Thrombosen und Thromboembolien, Ischämien (periphere, cardiale, cerebrale Durchblutungsstörungen), Herz- und Hirninfarkten, Herzrhythmusstörungen, Angina pectoris, Arteriosklerose, bei Gewebstransplantationen, Dermatosen wie Psoriasis, entzündliche Dermatosen, z.B. Ekzem, Dermatophyteninfektion, Infektionen der Haut durch Bakterien, Metastasen und zur Cytoprotection im Gastrointestinaltrakt geeignet.

Die erfindungsgemäßen disubstituierten (Chinolin-2-yl-methoxy)phenylessigsäure-Derivate können sowohl in der Humanmedizin als auch in der Veterinärmedizin angewendet werden.

Die pharmakologischen Wirkdaten der erfindungsgemäßen Substanzen werden durch folgende Methode bestimmt:
Als Maß für die 5-Lipoxygenase-Hemmung in vitro wurde die Freisetzung von Leukotrien B₄ (LTB₄) an polymorphkernigen Rattenleukozyten (PMN) nach Zugabe von Substanzen und Ca-Ionophor mittels reverse phase HPLC nach Borgeat, P. et al., Proc. Nat. Acad. Sci. 76, 2148-2152 (1979), bestimmt. Die in vivo-Aktivität der Verbindungen wurde mit dem Mäuseohr-Entzündungsmodell nach Young, J.M. et al., J. of Investigative Dermatology 82, 367-371, (1984) nachgewiesen.

Die neuen Wirkstoffe können in an sich bekannter Weise unter Verwendung inerter nichttoxischer, pharmazeutisch geeigneter Trägerstoffe oder Lösungsmittel in die üblichen Formulierungen überführt werden, wie Tabletten, Kapseln, Dragees, Pillen, Granulate, Aerosole, Sirupe, Emulsionen, Suspensionen und Lösungen. Hierbei soll die therapeutisch wirksame Verbindung jeweils in einer Konzentration von etwa 0,5 bis 90 Gew.-%, bevorzugt von 10 bis 70 Gew.-%, in der Zubereitung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nichttoxische organische Lösungsmittel wie Paraffine (z.B. Erdölfraktionen), pflanzliche Öle (z.B. Erdnuß/Sesamöl), Alkohole (z.B. Ethylalkohol, Glycerin), Glykole (z.B. Propylenglykol, Polyethylenglykol), feste Trägerstoffe, wie natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäureester, Polyoxyethylen-Fettalkohole-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation kann in üblicher Weise erfolgen, vorzugsweise oral oder parenteral, insbesondere perlingual oder intravenös. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,01 bis 5 mg/kg Körpergewicht, zur Erzielung wirksamer Ergebnisse zu verabreichen. Bei oraler Applikation beträgt die Dosierung im allgemeinen etwa 0,1 bis 200 mg/kg, vorzugsweise 1 bis 100 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und zu dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehreren Einzelgaben über den Tag zu verteilen.

### Herstellungsbeispiele

### Beispiel I (Formel II)

### 4-(Chinolin-2-yl-methoxy)phenylglyoxylsäuremethylester

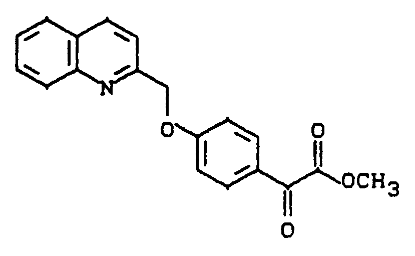

35 g (194 mmol) 4-Hydroxyphenylglyoxylsäuremethylester (Reg.-Nr. 38 250-16-7), 34,5 g (194 mmol) Chlormethylchinolin-Hydrochlorid (Aldrich) und 53,7 g (388 mmol) Kaliumcarbonat werden 20 h bei 50°C in 150 ml Dimethylformamid gerührt. Nach Abkühlen auf 25°C werden 250 ml Wasser zugegeben, das Produkt wird abgesaugt, getrocknet und aus Methanol umkristallisiert.
Ausbeute: 45 g (72% der Theorie)
Fp.: 105-108° C (Methanol)

### Beispiel 1 (Verbindungen der allgemeinen Formel I)

### 2-[4-(Chinolin-2-yl-methoxy)phenyl]-2-cyclopentylhydroxyessigsäuremethylester

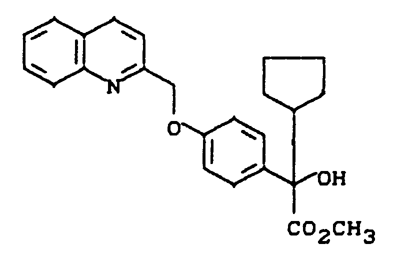

a) Grignard-Lösung
   Zu 0,8 g (33 mmol) Magnesium-Spänen in 50 ml Diethylether werden unter Schutzgas 4,9 g (33 mmol) Cyclopentylbromid so zugetropft, daß die Reaktionslösung siedet. Anschließend wird 1 h unter Rückfluß erwärmt.
b) 9,6 g (30 mmol) 4-(Chinolin-2-yl-methoxy)phenylglyoxylsäuremethylester werden unter Schutzgas in 50 ml Tetrahydrofuran gelöst und die Lösung auf 0°C gekühlt. Die bereitete Cyclopentylmagnesiumbromid-Lösung wird bei 0°C zugetropft. Nach Erwärmen auf 25°C wird 15 h gerührt. Das Reaktionsgemisch wird auf Eiswasser gegossen, mit Ammoniumchlorid angesäuert und mit Essigester extrahiert. Die organische Phase wird mit Natriumsulfat getrocknet, einrotiert und das Rohprodukt an Kieselgel 60 mit Cyclohexan/Essigester (3:1) chromatographiert.
   Ausbeute: 3 g (25,5% der Theorie)
   Festpunkt: 75-77°C (Ethanol)

### Beispiel 2

### 2-[4-(Chinolin-2-yl-methoxy)phenyl]-2-cyclopentylhydroxyessigsäure

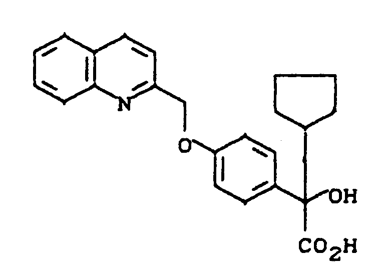

1,1 g (2,8 mmol) der Verbindung aus Beispiel 1 werden in 30 ml Methanol und 10 ml 2 normale Natronlauge bei 25°C gerührt. Nach Zugabe von 10 ml 2 normaler Salzsäure wird eingeengt, der Rückstand in Wasser aufgenommen und mit Essigester extrahiert. Nach Trocknen über Natriumsulfat wird eingeengt und der Rückstand mit Diisopropylether verrieben.
Ausbeute: 750 mg (71% der Theorie)
Fp.: 173-175°C (Methanol)

### Beispiel 3

### 2-[4-(Chinolin-2-yl-methoxy)phenyl)-2-cyclohexylhydroxyessigsäuremethylester

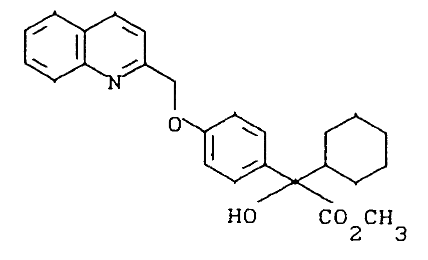

16,1 g (50 mmol) 4-(Chinolin-2-yl-methoxy)phenylglyoxalsäuremethylester werden mit 100 mmol Cyclohexylmagnesiumbromid wie in Beispiel 1 beschrieben umgesetzt.
Festpunkt: 126-128°C (Ethanol)
Ausbeute: 9,6 9 (47 % der Theorie)

### Beispiel 4

### 2-[4-Chinolin-2-yl-methoxy)phenyl]-2-cycloheptyl-hydroxyessigsäuremethylester

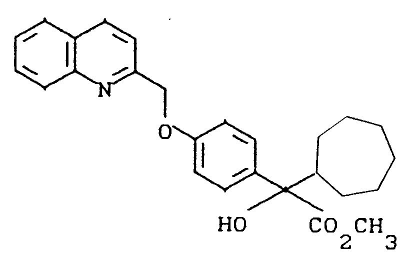

16,1 g (50 mmol) 4-(Chinolin-2-yl-methoxy)phenylglyoxalsäuremethylester werden mit 100 mmol Cycloheptylmagnesiumbromid wie in Beispiel 1 beschrieben umgesetzt.
Festpunkt: 102-103°C (Ethanol)
Ausbeute: 9,7 g (46 % der Theorie)

### Beispiel 5

### 2-[4-(Chinolin-2-yl-methoxy)phenyl]-2-cyclohexylhydroxy-essigsäure

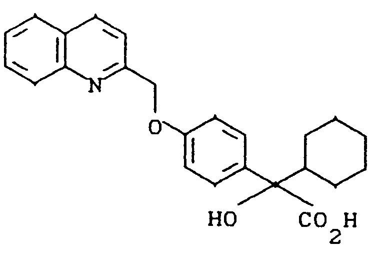

4,05 g (10 mmol) 2-[4-(Chinolin-2-yl-methoxy)phenyl]-2-cyclohexyl-hydroxyessigsäuremethylester werden analog Beispiel 2 verseift.
Festpunkt: 192-193°C (Ethanol)
Ausbeute: 2,6 g (70 % der Theorie)

### Beispiel 6

### 2-[4-(Chinolin-2-yl-methoxy)phenyl]-2-cycloheptyl-hydroxyessigsäure

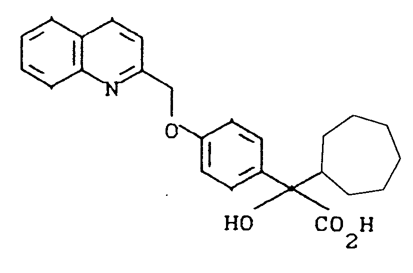

3 g (7,1 mmol) 2-[4-(Chinolin-2-yl-methoxy)-phenyl-2-cycloheptyl-hydroxyessigsäuremethylester werden analog Beispiel 2 verseift.
Festpunkt: 187-188°C (Ethanol)
Ausbeute: 2 g (69,5 % der Theorie)

## Patentansprüche (Patentansprüche für folgende(n) Vertragsstaat(en): AT, BE, CH, DE, DK, FR, GB, GR, IT, LI, LU, NL, SE)

1. Disubstituierte (Chinolin-2-yl-methoxy)phenylessigsäure-Derivate der allgemeinen Formel 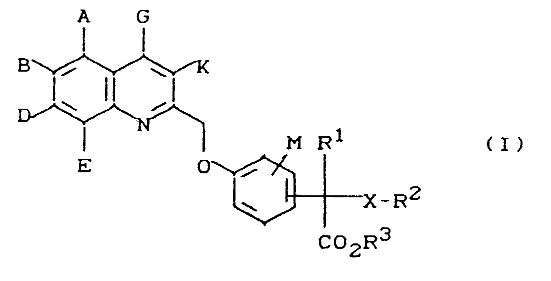 in welcher
A, B, D, E, G, K und M gleich oder verschieden sind und
- für Wasserstoff, Hydroxy, Halogen, Trifluormethyl, Trifluormethoxy oder Carboxy stehen,
- für geradkettiges oder verzweigtes Alkyl mit bis zu 10 Kohlenstoffatomen stehen, das gegebenenfalls durch Hydroxy oder Halogen substituiert ist,
- für geradkettiges oder verzweigtes Alkoxy oder Alkoxycarbonyl mit bis zu 10 Kohlenstoffatomen stehen, oder
- für Aryl mit 6 bis 10 Kohlenstoffatomen stehen, das gegebenenfalls durch Halogen, Nitro, Cyano oder durch geradkettiges oder verzweigtes Alkyl oder Alkoxy mit bis zu 8 Kohlenstoffatomen substituiert ist,
R¹
- für geradkettiges oder verzweigtes Alkyl mit bis zu 10 Kohlenstoffatomen steht, das gegebenenfalls durch Halogen oder durch Cycloalkyl mit 3 bis 8 Kohlenstoffatomen substituiert ist, oder
- für Cycloalkyl mit 3 bis 8 Kohlenstoffatomen steht, das gegebenenfalls durch Halogen oder geradkettiges oder verzweigtes Alkyl mit bis zu 8 Kohlenstoffatomen substituiert ist,
R²
- für Wasserstoff, geradkettiges oder verzweigtes Alkyl mit bis zu 10 Kohlenstoffatomen oder für Aryl mit 6 bis 10 Kohlenstoffatomen steht,
R³
- für Wasserstoff, geradkettiges oder verzweigtes Alkyl mit bis zu 10 Kohlenstoffatomen oder für Aryl mit 6 bis 10 Kohlenstoffatomen steht,
X
- für Sauerstoff oder Schwefel steht,
und deren Salze.

2. Disubstituierte (Chinolin-2-yl-methoxy)phenylessigsäure-Derivate der Formel nach Anspruch 1
worin
A, B, D, E, G, K und M gleich oder verschieden sind und
- für Wasserstoff, Fluor, Chlor, Trifluormethoxy oder Carboxy stehen,
- für geradkettiges oder verzweigtes Alkyl mit bis zu 8 Kohlenstoffatomen stehen, das gegebenenfalls durch Hydroxy, Fluor, Chlor oder Brom substituiert ist,
- für geradkettiges oder verzweigtes Alkoxy oder Alkoxycarbonyl mit bis zu 8 Kohlenstoffatomen stehen, oder
- für Phenyl stehen, das gegebenenfalls durch Fluor, Chlor, Brom, Nitro, Cyano oder durch geradkettiges oder verzweigtes Alkyl oder Alkoxy mit bis zu 6 Kohlenstoffatomen substituiert ist,
R¹
- für geradkettiges oder verzweigtes Alkyl mit bis zu 8 Kohlenstoffatomen steht, das gegebenenfalls durch Fluor, Chlor, Brom, Cyclopropyl, Cyclobutyl, Cyclopentyl, Cyclohexyl oder Cycloheptyl substituiert ist, oder
- für Cyclopropyl, Cyclobutyl, Cyclopentyl, Cyclohexyl oder Cycloheptyl steht, das gegebenenfalls durch Fluor, Chlor oder geradkettiges oder verzweigtes Alkyl mit bis zu 6 Kohlenstoffatomen substituiert ist,
R²
- für Wasserstoff, geradkettiges oder verzweigtes Alkyl mit bis zu 8 Kohlenstoffatomen oder für Phenyl steht,
R³
- für Wasserstoff, geradkettiges oder verzweigtes Alkyl mit bis zu 8 Kohlenstoffatomen oder Phenyl steht,
X
- für Sauerstoff oder Schwefel steht
und deren Salze.

3. Disubstituierte (Chinolin-2-yl-methoxy)-phenylessigsäure-Derivate der Formel nach Anspruch 1
worin
A, B, D, E, G, K und M gleich oder verschieden sind und
- für Wasserstoff, Fluor, Chlor, geradkettiges oder verzweigtes Alkyl oder Alkoxy mit bis zu 6 Kohlenstoffatomen stehen,
R¹
- für geradkettiges oder verzweigtes Alkyl mit bis zu 6 Kohlenstoffatomen steht, das gegebenenfalls durch Fluor, Cyclopropyl, Cyclobutyl, Cyclopentyl, Cyclohexyl oder Cycloheptyl substituiert ist, oder
- für Cyclopropyl, Cyclobutyl, Cyclopentyl, Cyclohexyl oder Cycloheptyl steht, das gegebenenfalls durch Fluor oder durch geradkettiges oder verzweigtes Alkyl mit bis zu 4 Kohlenstoffatomen substituiert ist,
R²
- für Wasserstoff oder für geradkettiges oder verzweigtes Alkyl mit bis zu 6 Kohlenstoffatomen oder für Phenyl steht,
R³
- für Wasserstoff oder für geradkettiges oder verweigtes Alkyl mit bis zu 6 Kohlenstoffatomen steht,
X
- für Sauerstoff steht
und deren Salze.

4. Disubstituierte (Chinolin-2-yl-methoxy)phenylessigsäure-Derivate nach Anspruch 1 bis 3 zur Bekämpfung von Krankheiten.

5. Verfahren zur Herstellung von disubstituierten (Chinolin-2-yl-methoxy)phenylessigsäure-Derivaten nach Anspruch 1 bis 3 dadurch gekennzeichnet, daß man Ketoester der allgemeinen Formel (II) 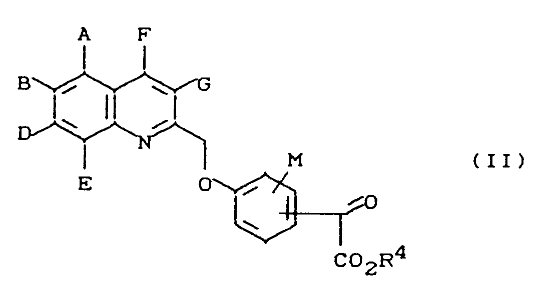 in welcher
A, B, D, E, G, K und M die oben angegegebene Bedeutung haben,
und
R⁴ - die oben angegebene Bedeutung von R³ hat, aber nicht für Wasserstoff steht,
zunächst mit Grignard- oder metallorganischen Verbindungen der allgemeinen Formeln (III) und (IIIa)
R¹-Y (III)
(R¹)ₙ-Y (III)
in welcher
R¹ die oben angegebene Bedeutung hat
n die Zahl 2 oder 3 bedeutet,
und
Y
- für den typischen Grignardrest Z-W steht,
worin
Z - Magnesium, Cadmium oder Zink bedeutet
und
W - Chlor, Brom oder Jod bedeutet,
oder
- für Lithium, Natrium, Magnesium, Aluminium, Cadmium oder Zink steht,
in inerten Lösemitteln reduziert und unter anschließender Abspaltung der Gruppe Y zu Verbindungen der allgemeinen Formel (Ia) 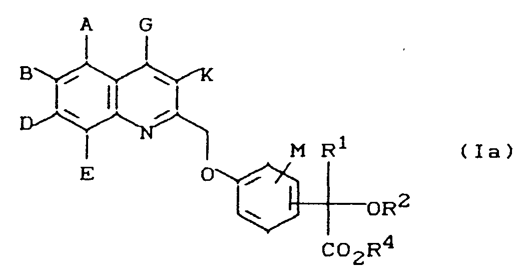 in welcher
A, B, D, E, G, K, M, R¹, R² und R⁴ die oben angegebene Bedeutung haben,
umsetzt,
und im Fall, daß X für Schwefel steht, die Verbindungen der Formel (Ia) nach üblicher Methode mit Thiolen der allgemeinen Formel (IV)
HS-R² (IV)
in welcher
R² die oben angegebene Bedeutung hat,
umsetzt,
und im Fall der Säuren in einem letzten Schritt die Ester alkalisch verseift.

6. Arzneimittel enthaltend disubstituierte (Chinolin-2-yl-methoxy)phenylessigsäure-Derivate nach Anspruch 1 bis 3.

7. Verfahren zur Herstellung eines Arzneimittels nach Anspruch 6, dadurch gekennzeichnet, daß man disubstituierte (Chinolin-2-yl-methoxy)phenylessigsäureDerivate nach Anspruch 1, gegebenenfalls mit Hilfe von üblichen Hilfs- und Trägerstoffen in eine geeignete Applikationsform bringt.

8. Verwendung von disubstituierten (Chinolin-2-yl-methoxy)phenylessigsäure-Derivaten nach Anspruch 1-3 zur Herstellung von Arzneimitteln.

9. Verwendung von disubstituierten (Chinolin-2-yl-methoxy)phenylessigsäure-Derivaten nach Anspruch 1-3 zur Herstellung von Lipoxygenasehemmern.

## Patentansprüche (Patentansprüche für folgende(n) Vertragsstaat(en): ES)

1. Verfahren zur Herstellung von disubstituierten (Chinolin-2-yl-methoxy)phenylessigsäure-Derivaten der allgemeinen Formel 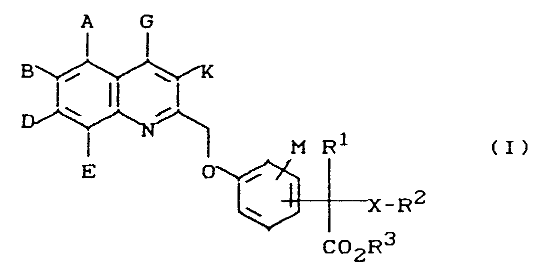 in welcher
A, B, D, E, G, K und M gleich oder verschieden sind und
- für Wasserstoff, Hydroxy, Halogen, Trifluormethyl, Trifluormethoxy oder Carboxy stehen,
- für geradkettiges oder verzweigtes Alkyl mit bis zu 10 Kohlenstoffatomen stehen, das gegebenenfalls durch Hydroxy oder Halogen substituiert ist,
- für geradkettiges oder verzweigtes Alkoxy oder Alkoxycarbonyl mit bis zu 10 Kohlenstoffatomen stehen, oder
- für Aryl mit 6 bis 10 Kohlenstoffatomen stehen, das gegebenenfalls durch Halogen, Nitro, Cyano oder durch geradkettiges oder verzweigtes Alkyl oder Alkoxy mit bis zu 8 Kohlenstoffatomen substituiert ist,
R¹
- für geradkettiges oder verzweigtes Alkyl mit bis zu 10 Kohlenstoffatomen steht, das gegebenenfalls durch Halogen oder durch Cycloalkyl mit 3 bis 8 Kohlenstoffatomen substituiert ist, oder
- für Cycloalkyl mit 3 bis 8 Kohlenstoffatomen steht, das gegebenenfalls durch Halogen oder geradkettiges oder verzweigtes Alkyl mit bis zu 8 Kohlenstoffatomen substituiert ist,
R²
- für Wasserstoff, geradkettiges oder verzweigtes Alkyl mit bis zu 10 Kohlenstoffatomen oder für Aryl mit 6 bis 10 Kohlenstoffatomen steht,
R³
- für Wasserstoff, geradkettiges oder verzweigtes Alkyl mit bis zu 10 Kohlenstoffatomen oder für Aryl mit 6 bis 10 Kohlenstoffatomen steht,
X
- für Sauerstoff oder Schwefel steht,
und deren Salze dadurch gekennzeichnet, daß man
Ketoester der allgemeinen Formel (II) 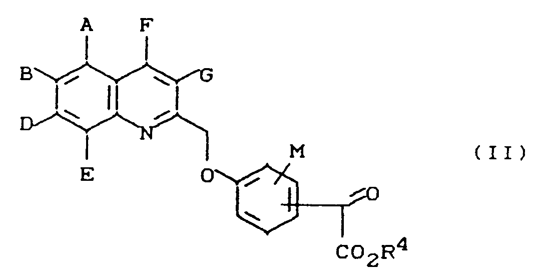 in welcher
A, B, D, E, G, K und M die oben angegegebene Bedeutung haben,
und
R⁴
- die oben angegebene Bedeutung von R³ hat, aber nicht für Wasserstoff steht,
zunächst mit Grignard- oder metallorganischen Verbindungen der allgemeinen Formeln (III) und (IIIa)
R¹-Y (III)
(R¹)ₙ-Y (III)
in welcher
R¹ die oben angegebene Bedeutung hat
n die Zahl 2 oder 3 bedeutet,
und
Y
- für den typischen Grignardrest Z-W steht,
worin
Z - Magnesium, Cadmium oder Zink bedeutet
und
W - Chlor, Brom oder Jod bedeutet,
oder
- für Lithium, Natrium, Magnesium, Aluminium, Cadmium oder Zink steht,
in inerten Lösemitteln reduziert und unter anschließender Abspaltung der Gruppe Y zu Verbindungen der allgemeinen Formel (Ia) 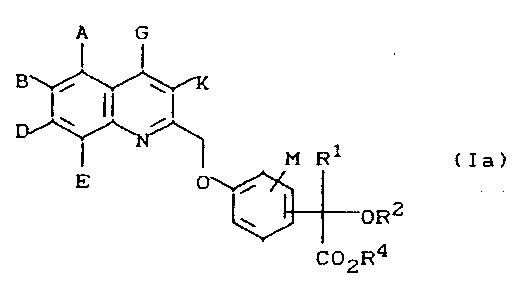 in welcher
A, B, D, E, G, K, M, R¹, R² und R⁴ die oben angegebene Bedeutung haben,
umsetzt,
und im Fall, daß X für Schwefel steht, die Verbindungen der Formel (Ia) nach üblicher Methode mit Thiolen der allgemeinen Formel (IV)
HS-R² (IV)
in welcher
R² die oben angegebene Bedeutung hat,
umsetzt,
und im Fall der Säuren in einem letzten Schritt die Ester alkalisch verseift.

## Claims (Claims for the following Contracting State(s): AT, BE, CH, DE, DK, FR, GB, GR, IT, LI, LU, NL, SE)

1. Disubstituted (quinolin-2-yl-methoxy)phenylacetic acid derivatives of the general formula 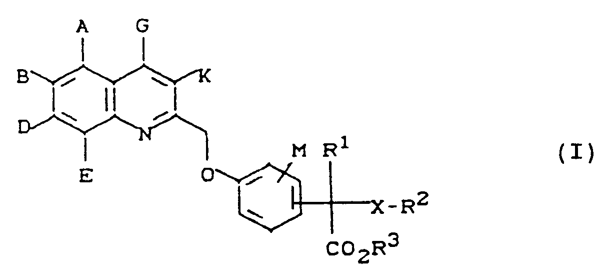 in which
A, B, D, E, G, K and M are identical or different and
- represent hydrogen, hydroxyl, halogen, trifluoromethyl, trifluoromethoxy or carboxyl,
- represent straight-chain or branched alkyl having up to 10 carbon atoms, which is optionally substituted by hydroxyl or halogen,
- represent straight-chain or branched alkoxy or alkoxycarbonyl having up to 10 carbon atoms, or
- represent aryl having 6 to 10 carbon atoms, which is optionally substituted by halogen, nitro, cyano or by straight-chain or branched alkyl or alkoxy having up to 8 carbon atoms,
R¹
- represents straight-chain or branched alkyl having up to 10 carbon atoms, which is optionally substituted by halogen or by cycloalkyl having 3 to 8 carbon atoms, or
- represents cycloalkyl having 3 to 8 carbon atoms, which is optionally substituted by halogen or straight-chain or branched alkyl having up to 8 carbon atoms,
R²
- represents hydrogen or straight-chain or branched alkyl having up to 10 carbon atoms, or represents aryl having 6 to 10 carbon atoms,
R³
- represents hydrogen or straight-chain or branched alkyl having up to 10 carbon atoms, or represents aryl having 6 to 10 carbon atoms and
X
- represents oxygen or sulphur,
and salts thereof.

2. Disubstituted (quinolin-2-yl-methoxy)phenylacetic acid derivatives of the formula according to Claim 1,
wherein
A, B, D, E, G, K and M are identical or different and
- represent hydrogen, fluorine, chlorine, trifluoromethoxy or carboxyl,
- represent straight-chain or branched alkyl having up to 8 carbon atoms, which is optionally substituted by hydroxyl, fluorine, chlorine or bromine,
- represent straight-chain or branched alkoxy or alkoxycarbonyl having up to 8 carbon atoms, or
- represent phenyl, which is optionally substituted by fluorine, chlorine, bromine, nitro, cyano or straight-chain or branched alkyl or alkoxy having up to 6 carbon atoms,
R¹
- represents straight-chain or branched alkyl having up to 8 carbon atoms, which is optionally substituted by fluorine, chlorine, bromine, cyclopropyl, cyclobutyl, cyclopentyl, cyclohexyl or cycloheptyl, or
- represents cyclopropyl, cyclobutyl, cyclopentyl, cyclohexyl or cycloheptyl, which is optionally substituted by fluorine, chlorine, or straight-chain or branched alkyl having up to 6 carbon atoms,
R²
- represents hydrogen or straight-chain or branched alkyl having up to 8 carbon atoms, or represents phenyl,
R³
- represents hydrogen, straight-chain or branched alkyl having up to 8 carbon atoms or phenyl and
X
- represents oxygen or sulphur,
and salts thereof.

3. Disubstituted (quinolin-2-yl-methoxy)phenylacetic acid derivatives of the formula according to Claim 1,
wherein
A, B, D, E, G, K and M are identical or different and
- represent hydrogen, fluorine, chlorine, or straight-chain or branched alkyl or alkoxy having up to 6 carbon atoms,
R¹
- represents straight-chain or branched alkyl having up to 6 carbon atoms, which is optionally substituted by fluorine, cyclopropyl, cyclobutyl, cyclopentyl, cyclohexyl or cycloheptyl, or
- represents cyclopropyl, cyclobutyl, cyclopentyl, cyclohexyl or cycloheptyl, which is optionally substituted by fluorine or by straight-chain or branched alkyl having up to 4 carbon atoms,
R²
- represents hydrogen, or represents straight-chain or branched alkyl having up to 6 carbon atoms, or represents phenyl,
R³
- represents hydrogen, or represents straight-chain or branched alkyl having up to 6 carbon atoms and
X
- represents oxygen,
and salts thereof.

4. Disubstituted (quinolin-2-yl-methoxy)phenylacetic acid derivatives according to Claims 1 to 3 for combating diseases.

5. Process for the preparation of disubstituted (quinolin-2-yl-methoxy)phenylacetic acid derivatives according to Claims 1 to 3, characterized in that
keto esters of the general formula (II) 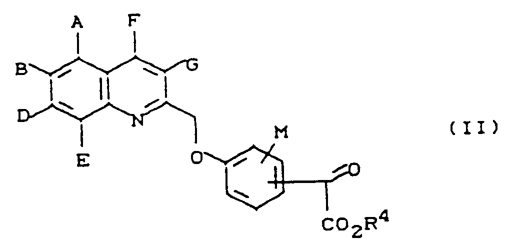 in which
A, B, D, E, G, K and M have the abovementioned meaning,
and
R⁴ - has the abovementioned meaning of R³ but does not represent hydrogen,
are first reduced with Grignard or organometallic compounds of the general formulae (III) and (IIIa)
R¹-Y (III)
(R¹)ₙ-Y (IIIa)
in which
R¹ - has the abovementioned meaning,
n denotes the number 2 or 3
and
Y
- represents the typical Grignard radical Z-W,
wherein
Z - denotes magnesium, cadmium or zinc and
W - denotes chlorine, bromine or iodine, or
- represents lithium, sodium, magnesium, aluminium, cadmium or zinc,
in inert solvents, and the products are converted, by subsequently splitting off the group Y, into compounds of the general formula (Ia) 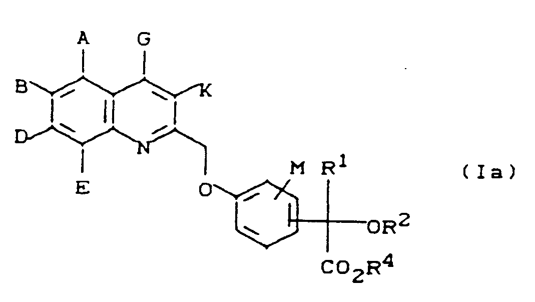 in which
A, B, D, E, G, K, M, R¹, R² and R⁴ have the abovementioned meaning,
and in the case where X represents sulphur, the compounds of the formula (Ia) are reacted with thiols of the general formula (IV)
HS-R² (IV)
in which
R² has the abovementioned meaning,
by the customary method, and in the case of the acids the esters are subjected to alkaline hydrolysis in a last step.

6. Medicaments containing disubstituted (quinolin-2-yl-methoxy)phenylacetic acid derivatives according to Claims 1 to 3.

7. Process for the preparation of a medicament according to Claim 6, characterized in that disubstituted (quinolin-2-yl-methoxy)phenylacetic acid derivatives according to Claim 1, are brought into a suitable administration form, if appropriate with the aid of customary auxiliaries and excipients.

8. Use of disubstituted (quinolin-2-yl-methoxy)phenylacetic acid derivatives according to Claims 1-3 for the preparation of medicaments.

9. Use of disubstituted (quinolin-2-yl-methoxy)phenylacetic acid derivatives according to Claims 1-3 for the preparation of lipoxygenase inhibitors.

## Claims (Claims for the following Contracting State(s): ES)

1. Process for the preparation of disubstituted (quinolin-2-yl-methoxy)phenylacetic acid derivatives of the general formula 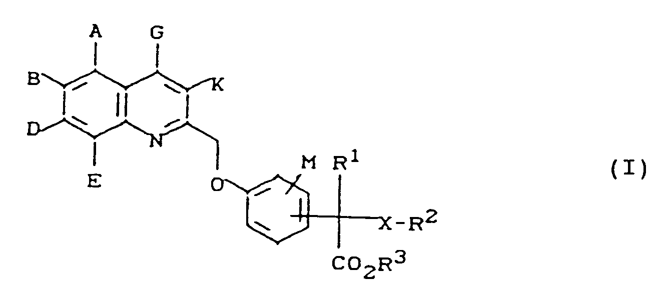 in which
A, B, D, E, G, K and M are identical or different and
- represent hydrogen, hydroxyl, halogen, trifluoromethyl, trifluoromethoxy or carboxyl,
- represent straight-chain or branched alkyl having up to 10 carbon atoms, which is optionally substituted by hydroxyl or halogen,
- represent straight-chain or branched alkoxy or alkoxycarbonyl having up to 10 carbon atoms, or
- represent aryl having 6 to 10 carbon atoms, which is optionally substituted by halogen, nitro, cyano or by straight-chain or branched alkyl or alkoxy having up to 8 carbon atoms,
R¹
- represents straight-chain or branched alkyl having up to 10 carbon atoms, which is optionally substituted by halogen or by cycloalkyl having 3 to 8 carbon atoms, or
- represents cycloalkyl having 3 to 8 carbon atoms, which is optionally substituted by halogen or straight-chain or branched alkyl having up to 8 carbon atoms,
R²
- represents hydrogen or straight-chain or branched alkyl having up to 10 carbon atoms, or represents aryl having 6 to 10 carbon atoms,
R³
- represents hydrogen or straight-chain or branched alkyl having up to 10 carbon atoms, or represents aryl having 6 to 10 carbon atoms and
X
- represents oxygen or sulphur,
and salts thereof, characterized in that
keto esters of the general formula (II) 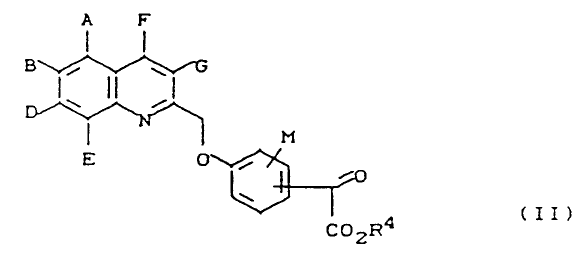 in which
A, B, D, E, G, K and M have the abovementioned meaning,
and
R⁴
- has the abovementioned meaning of R³ but does not represent hydrogen,
are first reduced with Grignard or organometallic compounds of the general formulae (III) and (IIIa)
R¹-Y (III)
(R¹)ₙ-Y (IIIa)
in which
R¹ - has the abovementioned meaning,
n denotes the number 2 or 3
and
Y
- represents the typical Grignard radical Z-W,
wherein
Z - denotes magnesium, cadmium or zinc and
W - denotes chlorine, bromine or iodine, or
- represents lithium, sodium, magnesium, aluminium, cadmium or zinc,
in inert solvents, and the products are converted, by subsequently splitting off the group Y, into compounds of the general formula (Ia) 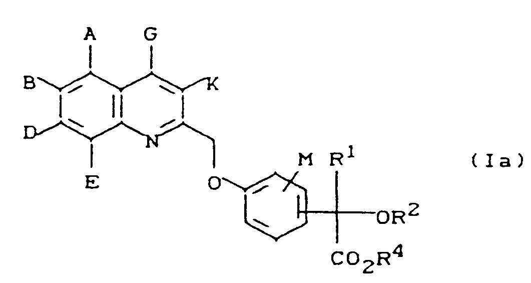 in which
A, B, D, E, G, K, M, R¹, R² and R⁴ have the abovementioned meaning,
and in the case where X represents sulphur, the compounds of the formula (Ia) are reacted with thiols of the general formula (IV)
HS-R² (IV)
in which
R² has the abovementioned meaning,
by the customary method, and in the case of the acids the esters are subjected to alkaline hydrolysis in a last step.

## Revendications (Revendications pour l'(les) Etat(s) contractant(s) suivant(s): AT, BE, CH, DE, DK, FR, GB, GR, IT, LI, LU, NL, SE)

1. Dérivés disubstitués de l'acide (quinoléin-2-yl-méthoxy)phénylacétique répondant à la formule générale 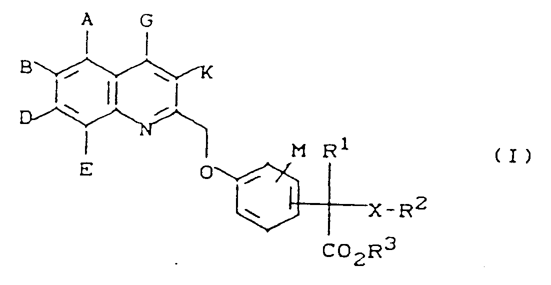 dans laquelle
A, B, D, E, G, K et M sont identiques ou différents et représentent
- un atome d'hydrogène, un groupe hydroxyle, un atome d'halogène, un groupe trifluorométhyle, un groupe trifluorométhoxy ou un groupe carboxyle,
- un groupe alkyle à chaîne droite ou ramifiée contenant jusqu'à 10 atomes de carbone, qui est éventuellement substitué par un groupe hydroxyle ou par un atome d'halogène,
- un groupe alcoxy ou un groupe alcoxycarbonyle à chaîne droite ou ramifiée contenant jusqu'à 10 atomes de carbone, ou
- représentent un groupe aryle contenant de 6 à 10 atomes de carbone, qui est éventuellement substitué par un atome d'halogène, par un groupe nitro, par un groupe cyano ou encore par un groupe alkyle ou par un groupe alcoxy à chaîne droite ou ramifiée contenant jusqu'à 8 atomes de carbone,
R¹
- représente un groupe alkyle à chaîne droite ou ramifiée contenant jusqu'à 10 atomes de carbone, qui est éventuellement substitué par un atome d'halogène ou par un groupe cycloalkyle contenant de 3 à 8 atomes de carbone, ou
- représente un groupe cycloalkyle contenant de 3 à 8 atomes de carbone, qui est éventuellement substitué par un atome d'halogène ou par un groupe alkyle à chaîne droite ou ramifiée contenant jusqu'à 8 atomes de carbone,
R²
- représente un atome d'hydrogène, un groupe alkyle à chaîne droite ou ramifiée contenant jusqu'à 10 atomes de carbone ou encore un groupe aryle contenant de 6 à 10 atomes de carbone,
R³
- représente un atome d'hydrogène, un groupe alkyle à chaîne droite ou ramifiée contenant jusqu'à 10 atomes de carbone ou encore un groupe aryle contenant de 6 à 10 atomes de carbone,
X
- représente un atome d'oxygène ou un atome de soufre,
ainsi que leurs sels.

2. Dérivés disubstitués de l'acide (quinoléin-2-yl-méthoxy)phénylacétique répondant à la formule selon la revendication 1,
dans lesquels
A, B, D, E, G, K et M sont identiques ou différents et représentent
- un atome d'hydrogène, un atome de fluor, un atome de chlore, un groupe trifluorométhoxy ou un groupe carboxyle,
- un groupe alkyle à chaîne droite ou ramifiée contenant jusqu'à 8 atomes de carbone, qui est éventuellement substitué par un groupe hydroxyle, par un atome de fluor, par un atome de chlore ou par un atome de brome,
- un groupe alcoxy ou un groupe alcoxycarbonyle à chaîne droite ou ramifiée contenant jusqu'à 8 atomes de carbone, ou
- représentent un groupe phényle qui est éventuellement substitué par un atome de fluor, par un atome de chlore, par un atome de brome, par un groupe nitro, par un groupe cyano ou encore par un groupe alkyle ou par un groupe alcoxy à chaîne droite ou ramifiée contenant jusqu'à 6 atomes de carbone,
R¹
- représente un groupe alkyle à chaîne droite ou ramifiée contenant jusqu'à 8 atomes de carbone, qui est éventuellement substitué par un atome de fluor, par un atome de chlore, par un atome de brome, par un groupe cyclopropyle, par un groupe cyclobutyle, par un groupe cyclopentyle, par un groupe cyclohexyle ou par un groupe cycloheptyle, ou
- représente un groupe cyclopropyle, un groupe cyclobutyle, un groupe cyclopentyle, un groupe cyclohexyle ou un groupe cycloheptyle qui est éventuellement substitué par un atome de fluor, par un atome de chlore ou par un groupe alkyle à chaîne droite ou ramifiée contenant jusqu'à 6 atomes de carbone,
R²
- représente un atome d'hydrogène, un groupe alkyle à chaîne droite ou ramifiée contenant jusqu'à 8 atomes de carbone ou encore un groupe phényle,
R³
- représente un atome d'hydrogène, un groupe alkyle à chaîne droite ou ramifiée contenant jusqu'à 8 atomes de carbone ou encore un groupe phényle,
X
- représente un atome d'oxygène ou un atome de soufre,
ainsi que leurs sels.

3. Dérivés disubstitués de l'acide (quinoléin-2-yl-méthoxy)phénylacétique répondant à la formule selon la revendication 1
dans lesquels
A, B, D, E, G, K et M sont identiques ou différents et représentent
- un atome d'hydrogène, un atome de fluor, un atome de chlore, un groupe alkyle ou un groupe alcoxy à chaîne droite ou ramifiée contenant jusqu'à 6 atomes de carbone,
R¹
- représente un groupe alkyle à chaîne droite ou ramifiée contenant jusqu'à 6 atomes de carbone, qui est éventuellement substitué par un atome de fluor, par un groupe cyclopropyle, par un groupe cyclobutyle, par un groupe cyclopentyle, par un groupe cyclohexyle ou par un groupe cycloheptyle, ou bien
- représente un groupe cyclopropyle, un groupe cyclobutyle, un groupe cyclopentyle, un groupe cyclohexyle ou un groupe cycloheptyle, qui est éventuellement substitué par un atome de fluor ou par un groupe alkyle à chaîne droite ou ramifiée contenant jusqu'à 4 atomes de carbone,
R²
- représente un atome d'hydrogène ou un groupe alkyle à chaîne droite ou ramifiée contenant jusqu'à 6 atomes de carbone ou encore un groupe phényle,
R³
- représente un atome d'hydrogène ou un groupe alkyle à chaîne droite ou ramifiée contenant jusqu'à 6 atomes de carbone,
X
- représente un atome d'oxygène,
ainsi que leurs sels.

4. Dérivés disubstitués de l'acide (quinoléin-2-yl-méthoxy)phénylacétique selon les revendications 1 à 3, pour lutter contre des maladies.

5. Procédé pour la préparation de dérivés disubstitués de l'acide (quinoléin-2-yl-méthoxy)phénylacétique selon les revendications 1 à 3,
caractérisé en ce qu'on réduit d'abord des esters cétoniques répondant à la formule générale (II) 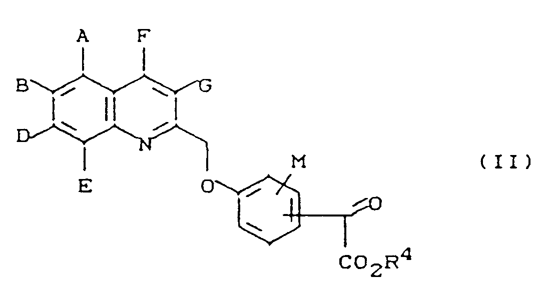 dans laquelle
A, B, D, E, G, K et M ont la signification indiquée ci-dessus, et
R⁴ - a la signification indiquée ci-dessus pour R³, mais ne représente pas un atome d'hydrogène,
avec des composés de Grignard ou organométalliques répondant aux formules générales (III) et (IIIa)
R¹-Y (III)
(R¹)ₙ-Y (IIIa)
dans lesquelles
R¹ a la signification indiquée ci-dessus,
n représente le nombre 2 ou 3,
et
Y
- représente le radical de Grignard spécifique Z-W,
où
Z - représente le magnésium, le cadmium ou le zinc
et
W - représente un atome de chlore, un atome de brome ou un atome d'iode,
ou
- représente le lithium, le sodium, le magnésium, l'aluminium, le cadmium ou le zinc,
dans des solvants inertes et, avec séparation ultérieure du groupe Y, on met à réagir pour obtenir des composés répondant à la formule générale (Ia) 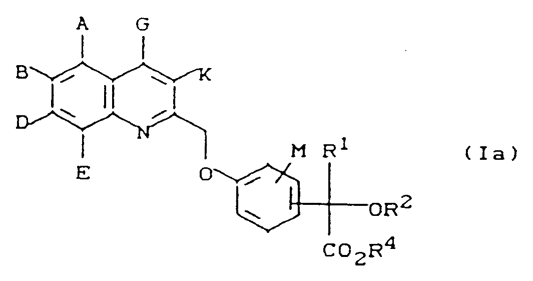 dans laquelle
A, B, D, E, G, K, M, R¹, R² et R⁴ ont la signification indiquée ci-dessus,
et, dans le cas où X représente un atome de soufre, on fait réagir les composés de formule (Ia) conformément à des procédés habituels avec des thiols répondant à la formule générale (IV)
HS-R² (IV)
dans laquelle
R² a la signification indiquée ci-dessus,
et, dans le cas des acides, dans une dernière étape, on saponifie les esters par voie alcaline.

6. Médicament contenant des dérivés disubstitués de l'acide (quinoléin-2-yl-méthoxy)phénylacétique selon les revendications 1 à 3.

7. Procédé pour la préparation d'un médicament selon la revendication 6, caractérisé en ce qu'on amène, dans une forme d'application appropriée, des dérivés disubstitués de l'acide (quinoléin-2-yl-méthoxy)phénylacétique selon la revendication 1, éventuellement à l'aide de substances auxiliaires et de support habituelles.

8. Utilisation de dérivés disubstitués de l'acide (quinoléin-2-yl-méthoxy)phénylacétique selon les revendications 1 à 3 pour la préparation de médicaments.

9. Utilisation de dérivés disubstîtués de l'acide (quinoléin-2-yl-méthoxy)phénylacétique selon les revendications 1 à 3 pour la préparation d'inhibiteurs de la lipoxygénase.

## Revendications (Revendications pour l'(les) Etat(s) contractant(s) suivant(s): ES)

1. Procédé pour la préparation de dérivés disubstitués de l'acide (quinoléin-2-yl-méthoxy)phénylacétique répondant à la formule générale 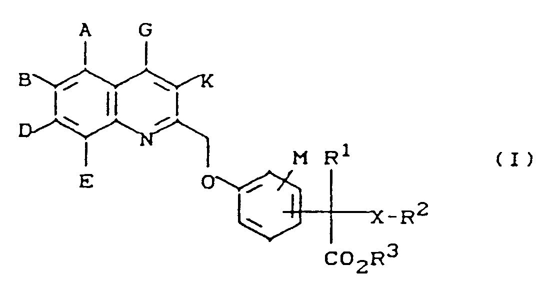 dans laquelle
A, B, D, E, G, K et M sont identiques ou différents et représentent
- un atome d'hydrogène, un groupe hydroxyle, un atome d'halogène, un groupe trifluorométhyle, un groupe trifluorométhoxy ou un groupe carboxyle,
- un groupe alkyle à chaîne droite ou ramifiée contenant jusqu'à 10 atomes de carbone, qui est éventuellement substitué par un groupe hydroxyle ou par un atome d'halogène,
- un groupe alcoxy ou un groupe alcoxycarbonyle à chaîne droite ou ramifiée contenant jusqu'à 10 atomes de carbone, ou
- représentent un groupe aryle contenant de 6 à 10 atomes de carbone, qui est éventuellement substitué par un atome d'halogène, par un groupe nitro, par un groupe cyano ou encore par un groupe alkyle ou par un groupe alcoxy à chaîne droite ou ramifiée contenant jusqu'à 8 atomes de carbone,
R¹
- représente un groupe alkyle à chaîne droite ou ramifiée contenant jusqu'à 10 atomes de carbone, qui est éventuellement substitué par un atome d'halogène ou par un groupe cycloalkyle contenant de 3 à 8 atomes de carbone, ou
- représente un groupe cycloalkyle contenant de 3 à 8 atomes de carbone, qui est éventuellement substitué par un atome d'halogène ou par un groupe alkyle à chaîne droite ou ramifiée contenant jusqu'à 8 atomes de carbone,
R²
- représente un atome d'hydrogène, un groupe alkyle à chaîne droite ou ramifiée contenant jusqu'à 10 atomes de carbone ou encore un groupe aryle contenant de 6 à 10 atomes de carbone,
R³
- représente un atome d'hydrogène, un groupe alkyle à chaîne droite ou ramifiée contenant jusqu'à 10 atomes de carbone ou encore un groupe aryle contenant de 6 à 10 atomes de carbone,
X
- représente un atome d'oxygène ou un atome de soufre,
et de leurs sels,
caractérisé en ce qu'on réduit d'abord des esters cétoniques répondant à la formule générale (II) 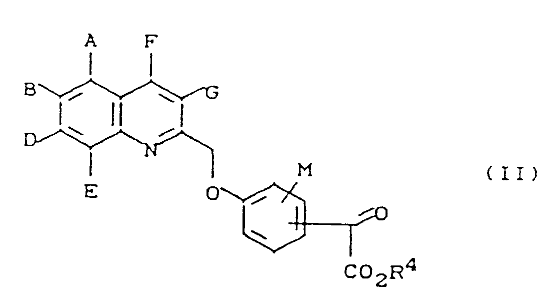 dans laquelle
A, B, D, E, G, K et M ont la signification indiquée ci-dessus, et
R⁴ - a la signification indiquée ci-dessus pour R³, mais ne représente pas un atome d'hydrogène,
avec des composés de Grignard ou organométalliques répondant aux formules générales (III) et (IIIa)
R¹-Y (III)
(R¹)ₙ-Y (IIIa)
dans lesquelles
R¹ a la signification indiquée ci-dessus,
n représente le nombre 2 ou 3,
et
Y
- représente le radical de Grignard spécifique Z-W,
où
Z - représente le magnésium, le cadmium ou le zinc
et
W - représente un atome de chlore, un atome de brome ou un atome d'iode,
ou
- représente le lithium, le sodium, le magnésium, l'aluminium, le cadmium ou le zinc,
dans des solvants inertes et, avec séparation ultérieure du groupe Y, on met à réagir pour obtenir des composés répondant à la formule générale (Ia) 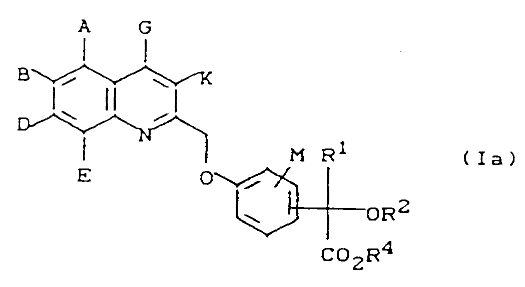 dans laquelle
A, B, D, E, G, K, M, R¹, R² et R⁴ ont la signification indiquée ci-dessus,
et, dans le cas où X représente un atome de soufre, on fait réagir les composés de formule (Ia) conformément à des procédés habituels avec des thiols répondant à la formule générale (IV)
HS-R² (IV)
dans laquelle
R² a la signification indiquée ci-dessus,
et, dans le cas des acides, dans une dernière étape, on saponifie les esters par voie alcaline.
